# EUROPEAN PATENT SPECIFICATION

(11) **EP 1 888 577 B1**
(45) Date of publication and mention of the grant of the patent: **12.11.2008**
(21) Application number: 06710597.3
(22) Date of filing: 24.02.2006
(51) Int. Cl.: C07D 471/04, A61K 31/519

(54) **OCAPERIDONE SALTS AND PHARMACEUTICAL COMPOSITIONS CONTAINING THE SAME**
OCAPERIDONSALZE UND PHARMAZEUTISCHE ZUSAMMENSETZUNGEN, DIE DIESE ENTHALTEN
SEL D'OCAPERIDONE ET COMPOSITION PHARMACEUTIQUE LE CONTENANT.

(30) Priority: 24.02.2005 EP 05290428
(43) Date of publication of application: 20.02.2008
(73) Proprietor: Janssen Pharmaceutica NV, 2340 Beerse (BE)
(72) Inventor: BIONDI, Stefano, 37057 Verona (IT); DEMAILLY, Arnold, F-61800 Mulhouse (FR); MONDADORI, Cesare, CH-4153 Reinach (CH); PAPARIN, Jean-Laurent, F-68720 Zillisheim (FR)
(74) Representative: Quaghebeur, Luc
(86) International application number: PCT/IB2006/000688
(87) International publication number: WO 2006/090285

(56) References cited:
- EP-A- 0 453 042
- WO-A-94/25460
- WO-A-20/04094414
- US-A1- 2003 191 141
- JOTTIER W I ET AL: "STRUCTURE OF THE ANTIPSYCHOTIC DRUG 3-ä2-Ä4-(6-FLUORO-1,2-BENZISOXAZO L-3-YL)-1-PIPERIDINYLÜETHYLü-2,9-DIMETHYL- 4H-PYRIDOÄ1,2-AÜPYRIMIDIN- 4-ONE (OCAPERIDONE)" ACTA CRYSTALLOGRAPHICA SECTION C. CRYSTAL STRUCTURE COMMUNICATIONS, MUNKSGAARD, COPENHAGEN, DK, vol. C48, no. 10, 1992, pages 1827-1830, XP009054985 ISSN: 0108-2701

## Description

The invention relates to new salts of ocaperidone and uses thereof, particularly in the pharmaceutical industry. The invention discloses specific salts of ocaperidone having increased water solubilities, as well as therapeutic methods by administering said salts, in particular for treating various diseases of the central or peripheral nervous system, especially central nervous system. It further deals with pharmaceutical compositions comprising said salts and methods for preparing the same.

European patent n° 0453042 describes the 3-[2-[4-(6-fluoro-1,2-benzisoxazol-3-yl)-1-piperidinyl]ethyl]-2,9-dimethyl-pyrido-[1,2-a] pyrimidin-4-one also known as ocaperidone, a potent dopaminergic compound having particularly interesting antipsychotic properties. It has been described as an antagonist of neurotransmitters and in particular dopamine and serotonin. Therapeutic indications for using ocaperidone therefore are mainly in the CNS area, particularly as a potent antipsychotic agent and more specifically for treating acute psychoses including schizophrenia, obsessive compulsive disorder (OCD), bipolar depression, and Tourette syndrome. Indeed, its combined dopamine-serotonin antagonism properties are especially interesting as they offer relief of both the positive and negative symptoms of schizophrenia.

Ocaperidone presents the following formula (I):

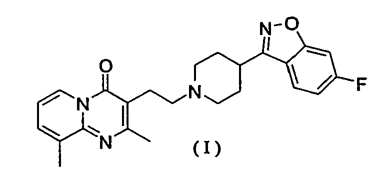

In view of its useful pharmacological properties, the subject compound is a candidate as a pharmacologically active ingredient. Water solubility is an essential parameter for the absorption of drugs and usually readily solubilized compounds are better absorbed by passive diffusion in the gastro-intestinal track. Unfortunately, ocaperidone as a free base form is poorly soluble in water (0.0007g/100ml) and also in acid solution (0.25 g/100ml at pH = 1.3), which may result in variability in bioavailability. Acid addition salts of ocaperidone due to their increased water solubility may be advantageous over the corresponding base form in the preparation of pharmaceutical compositions.

The Applicant has now found that particular salts of ocaperidone can be obtained in well defined reproducible amorphous and/or crystalline forms that especially exhibit valuable characteristics for formulation. The present invention deals with specific salts of ocaperidone which show very interesting water solubilites, in particular of at least 2.5 mg/ml. The use of such a salt form in the formulation of ocaperidone may give rise to a reduction in the variability of bioavailability from an improvement in absorption and less variability in plasma levels.

The present invention relates to unexpected soluble (1:1) salts of ocaperidone in amorphous and/or crystalline forms, including all polymorphs forms of the salts. The salt of the present invention presents an organic acid moiety.

More specifically, the ocaperidone salt according to the invention presents an acid moiety which is selected from pyroglutamic acid, N-(2-carboxyphenyl)-glycine acid, diglycolic acid, orotic acid, galactaric acid, nicotinic acid and hippuric acid. In a preferred embodiment, the acid moiety is selected from pyroglutamic acid, nicotinic acid and hippuric acid.

According to the invention, the acid moiety, in particular pyroglutamic acid, is in the D- or L- form, if existent, or mixture thereof; more particularly the L-form, most preferably L-pyroglutamic acid form.

Ocaperidone salts presenting an acid moiety selected from succinic acid, fumaric acid, tartaric acid, pyroglutamic acid, N-(2-carboxyphenyl)-glycine acid, diglycolic acid, orotic acid, galactaric acid, nicotinic acid and hippuric acid were made for comparison. As shown in more detail below, the ocaperidone salt solubility study shows that L-pyroglutamic acid addition salt is respectively 31.2 times more soluble than the succinic acid addition salt which is again some 357 times more soluble than the ocaperidone free base form.

The compound according to the present invention may be prepared by various methods known to those skilled in the art. However, the ocaperidone salt of the invention is generally prepared by dissolution of ocaperidone and the organic acid as defined above, preferably in stoichiometric proportion, and advantageously in an organic solvent, for example, dichloromethane, methanol, ethanol, tetrahydrofuran, ethyl acetate, ethers or mixture thereof. The temperature of the process can vary over a wide range, preferably between 0°C and the boiling temperature of the solvents used. When the salt is prepared, after a time comprised between a few seconds and several days, a crystalline product precipitates which can be isolated or collected by filtration, then can be washed with a cold (i.e. from 0°C to 25°C) organic solvent (preferably the same used for the crystallization step) and finally dried, preferably under vacuum.
In the crystallisation process according to the invention, it is possible to use the compound of Formula (I), in particular as free base form, obtained by any process. Advantageously, the compound of Formula (I) as a free base form is obtained by the preparation process described in patent specification EP 0453042.

In a particular embodiment of the process according to the invention, the concentration of ocaperidone in the solvent is preferably from 10-200 g/litre. The concentration of ocaperidone is preferably close to saturation.

It should be understood that other methods of producing the compound of the invention may be designed by the skilled person, based on common general knowledge and following guidance contained in this application.

According to a particular embodiment, the present invention relates to L-pyroglutamic acid addition salt of Formula (II). The salt of Formula (II) can be in amorphous and/or crystalline forms including all polymorphs of this compound.
Formula (II) is as follows :

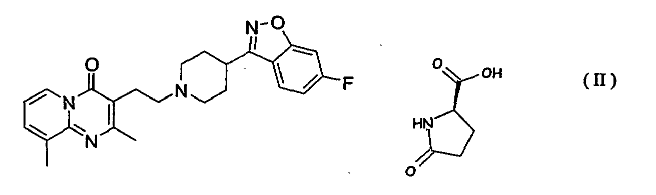

More specifically, the present invention relates to the crystalline form of the compound of formula (II), characterised by the powder X-ray diffraction diagram (see table (1) below), measured using an X-ray diffractometer (DRX) Rigaku Miniflex (copper anticathode) and expressed in terms of inter-planar distance d (A), Bragg's angle 2 theta (°), intensity (I) and relative intensity (expressed as a percentage of the most intense ray =Io):

**Table (1)**

| Peak N° | 2 theta (°) | d (Å) | Intensity I | I/Io |
|---|---|---|---|---|
| 1 | 5.260 | 16.7863 | 845 | 8 |
| 2 | 11.040 | 8.0074 | 546 | 5 |
| 3 | 12.000 | 7.3689 | 990 | 9 |
| 4 | 13.380 | 6.6118 | 3917 | 34 |
| 5 | 15.000 | 5.9012 | 1196 | 11 |
| 6 | 16.140 | 5.4868 | 3023 | 26 |
| 7 | 17.960 | 4.9347 | 1637 | 14 |
| 8 | 19.040 | 4.6572 | 2551 | 22 |
| 9 | 20.160 | 4.4009 | 2768 | 24 |
| 10 | 21.440 | 4.1409 | 11687 | 100 |
| 11 | 22.360 | 3.9726 | 4965 | 43 |
| 12 | 23.280 | 3.8176 | 4877 | 42 |
| 13 | 23.980 | 3.7078 | 2645 | 23 |
| 14 | 25.000 | 3.5588 | 1761 | 16 |
| 15 | 26.600 | 3.3482 | 3955 | 34 |
| 16 | 28.160 | 3.1662 | 2479 | 22 |
| 17 | 28.480 | 3.1313 | 2197 | 19 |
| 18 | 29.980 | 2.9780 | 1951 | 17 |
| 19 | 31.640 | 2.8254 | 1843 | 16 |
| 20 | 32.620 | 2.7427 | 1685 | 15 |
| 21 | 33.560 | 2.6680 | 1044 | 9 |
| 22 | 34.180 | 2.6210 | 1034 | 9 |
| 23 | 35.420 | 2.5321 | 1312 | 12 |
| 24 | 36.160 | 2.4819 | 1260 | 11 |
| 25 | 36.960 | 2.4300 | 939 | 9 |
| 26 | 37.800 | 2.3779 | 1095 | 10 |
| 27 | 38.980 | 2.3086 | 1130 | 10 |
| 28 | 39.440 | 2.2828 | 1028 | 9 |
| 29 | 40.440 | 2.2286 | 1006 | 9 |
| 30 | 41.589 | 2.1701 | 1162 | 10 |
| 31 | 43.080 | 2.0979 | 1073 | 10 |
| 32 | 44.120 | 2.0509 | 1462 | 13 |
| 33 | 44.660 | 2.0273 | 1292 | 12 |
| 34 | 45.640 | 1.9860 | 1115 | 10 |
| 35 | 46.860 | 1.9371 | 1488 | 13 |
| 36 | 47.940 | 1.8960 | 1014 | 9 |
| 37 | 48.300 | 1.8827 | 1016 | 9 |
| 38 | 49.140 | 1.8524 | 1273 | 11 |
| 39 | 49.700 | 1.8329 | 1160 | 10 |
| 40 | 51.020 | 1.7885 | 1042 | 9 |
| 41 | 52.960 | 1.7275 | 1056 | 10 |

In a similar manner, the nicotinic acid addition salt of ocaperidone (Formula (III)) was also prepared. The salt of Formula (III) can be in amorphous and/or crystalline forms including all polymorphs of this compound.
Formula (III) is as follows :

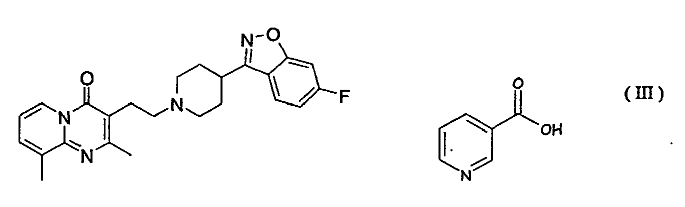

More specifically, the crystalline form of compound of formula (III) is characterised by the powder X-ray diffraction diagram (see table (2) below), measured using an X-ray diffractometer (DRX) Rigaku Miniflex (copper anticathode) and expressed in terms of inter-planar distance d (Å), Bragg's angle 2 theta (°), intensity and relative intensity (expressed as a percentage of the most intense ray):

**Table (2)**

| Peak N° | 2 theta (°) | d (Å) | Intensity I | I/Io |
|---|---|---|---|---|
| 1 | 11.060 | 7.9929 | 998 | 12 |
| 2 | 11.880 | 7.4430 | 927 | 11 |
| 3 | 12.520 | 7.0639 | 2480 | 29 |
| 4 | 13.300 | 6.6514 | 4841 | 57 |
| 5 | 14.720 | 6.0128 | 1226 | 15 |
| 6 | 16.420 | 5.3939 | 3993 | 47 |
| 7 | 17.360 | 5.1039 | 1209 | 15 |
| 8 | 18.900 | 4.6913 | 1911 | 23 |
| 9 | 20.120 | 4.4095 | 2031 | 24 |
| 10 | 21.240 | 4.1795 | 6837 | 80 |
| 11 | 22.180 | 4.0044 | 8591 | 100 |
| 12 | 23.100 | 3.8470 | 2534 | 30 |
| 13 | 23.940 | 3.7139 | 3596 | 42 |
| 14 | 25.020 | 3.5560 | 1512 | 18 |
| 15 | 25.660 | 3.4687 | 1318 | 16 |
| 16 | 26.420 | 3.3706 | 2100 | 25 |
| 17 | 27.600 | 3.2291 | 1737 | 21 |
| 18 | 28.480 | 3.1313 | 1285 | 15 |
| 19 | 29.600 | 3.0153 | 1288 | 15 |
| 20 | 30.040 | 2.9722 | 1236 | 15 |
| 21 | 31.360 | 2.8500 | 984 | 12 |
| 22 | 32.340 | 2.7658 | 959 | 12 |
| 23 | 33.980 | 2.6360 | 754 | 9 |
| 24 | 35.240 | 2.5446 | 1097 | 13 |
| 25 | 36.060 | 2.4886 | 736 | 9 |
| 26 | 37.860 | 2.3743 | 792 | 10 |
| 27 | 38.660 | 2.3270 | 744 | 9 |
| 28 | 39.460 | 2.2816 | 907 | 11 |
| 29 | 40.460 | 2.2275 | 941 | 11 |
| 30 | 41.560 | 2.1711 | 852 | 10 |
| 31 | 43.000 | 2.1016 | 1416 | 17 |
| 32 | 44.360 | 2.0403 | 967 | 12 |
| 33 | 45.500 | 1.9918 | 998 | 12 |
| 34 | 46.660 | 1.9450 | 1343 | 16 |
| 35 | 48.400 | 1.8790 | 859 | 10 |
| 36 | 49.340 | 1.8454 | 1029 | 12 |
| 37 | 54.940 | 1.6698 | 1401 | 17 |

In a similar manner, the hippuric acid addition salt of ocaperidone (Formula (IV)) was also prepared. The salt of Formula (IV) can be in amorphous and/or crystalline forms including all polymorphs of this compound.
Formula (IV) is as follows :

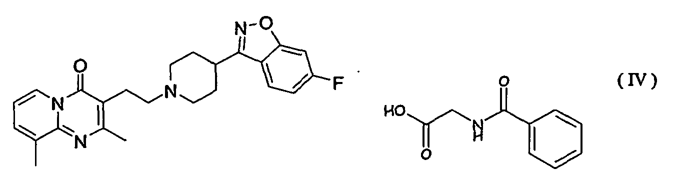

Accordingly, the N-(2-carboxyphenyl)-glycine acid, diglycolic acid, orotic acid, or galactaric acid addition salts of ocaperidone were also prepared. Said salts can be in amorphous and/or crystalline forms including all polymorphs of this compound.

The present invention also relates to pharmaceutical compositions comprising at least one ocaperidone salt as defined above in a pharmaceutically acceptable vehicle or support, optionally in association with another active agent.

The pharmaceutical composition is more particularly intended to treat diseases of the central or peripheral nervous system, especially central nervous diseases, including psychosis. The present salt is particularly effective in treating acute psychoses including schizophrenia, obsessive compulsive disorder (OCD), bipolar depression, anxiety, mania, and Tourette syndrome. The present salt is more particularly effective in treating schizophrenia, including positive and negative symptoms of schizophrenia, e.g. anergy, apathy, social withdrawal and depressive mood, and also appear to reduce the incidence of extrapyramidal side-effects during maintenance therapy with classical neuroleptics, i.e. dopamine antagonists.

The present invention also relates to the use of an ocaperidone salt as defined above, for the preparation of a pharmaceutical composition for the treatment of diseases of the central or peripheral nervous system, in particular for the treatment of diseases as specified above.

The present invention also includes methods of treating diseases of the central or peripheral nervous system, in particular for the treatment of diseases as specified above, comprising the administration to a subject in need thereof of an effective amount of a ocaperidone salt as defined above.

As indicated above, a further object of this invention relates to a pharmaceutical composition comprising at least one ocaperidone salt as defined above, and a pharmaceutically acceptable vehicle or support.

The compound may be formulated in various forms, including solid and liquid forms, such as tablets, gels, syrups, powders, aerosols, etc. The ocaperidone salt can be formulated either in crystalline and/or amorphous forms.

The compositions of this invention may contain physiologically acceptable diluents, fillers, lubricants, excipients, solvents, binders, stabilizers, and the like. Diluents that may be used in the compositions include but are not limited to dicalcium phosphate, calcium sulphate, lactose, cellulose, kaolin, mannitol, sodium chloride, dry starch, powdered sugar and for prolonged release tablet-hydroxy propyl methyl cellulose (HPMC). The binders that may be used in the compositions include but are not limited to starch, gelatin and fillers such as sucrose, glucose, dextrose and lactose.
Natural and synthetic gums that may be used in the compositions include but are not limited to sodium alginate, ghatti gum, carboxymethyl cellulose, methyl cellulose, polyvinyl pyrrolidone and veegum. Excipients that may be used in the compositions include but are not limited to microcrystalline cellulose, calcium sulfate, dicalcium phosphate, starch, magnesium stearate, lactose, and sucrose. Stabilizers that may be used include but are not limited to polysaccharides such as acacia, agar, alginic acid, guar gum and tragacanth, amphotsics such as gelatin and synthetic and semi-synthetic polymers such as carbomer resins, cellulose ethers and carboxymethyl chitin.
Solvents that may be used include but are not limited to Ringers solution, water, distilled water, dimethyl sulfoxide to 50% in water, propylene glycol (neat or in water), phosphate buffered saline, balanced salt solution, glycol and other conventional fluids.

The dosages and dosage regimen in which the salts of the invention are administered will vary according to the dosage form, mode of administration, the condition being treated and particulars of the patient being treated. Accordingly, optimal therapeutic concentrations will be best determined at the time and place through experimentation.

The ocaperidone salt according to the invention can also be used enterally. The required dose can be administered in one or more portions. For oral administration, suitable forms are, for example, tablets, gels, aerosols, pills, dragees, syrups, suspensions, emulsions, solutions, powders and granules; a preferred method of administration consists in using a suitable form containing approximately from 0.01 mg to about 1 mg of active substance per subject and day.

The compound according to the invention can also be administered parenterally in the form of solutions or suspensions for intradermal, intravenous infusions or intramuscular injections. In case in intravenous infusion, the compound according to the invention is generally administered at the rate of about 80 ng to 1000 ng per day (and to be infused within about an hour) per kg of body weight; a preferred method of administration consists of using i.v. solutions containing approximately from 0.25 µg to 3 µg of active substance per ml. In case of the intramuscular injection, the compound according to the invention is generally administered in a dose of approximately 0.6 µg to 5 µg per day per kg of body weight, a preferred method of administration would be to inject a volume of 1 ml containing approximately 30 µg to 300 µg.

For the compound of this invention, the dose to be administered, whether a single dose, multiple dose, or a daily dose, will of course vary with the chosen route of administration, the size of the recipient, the type of disease and the nature of the patient's condition. The dosage to be administered is not subject to definite bounds, but it will usually be an effective amount, or the equivalent on a molar basis of the pharmacologically active free form produced from a dosage formulation upon the metabolic release of the active drug to achieve its desired pharmacological and physiological effects. A doctor skilled in the art for treating the disease will be able to ascertain, without undue experimentation, appropriate protocols for the effective administration of the compound of this present invention, such as by referring to the earlier published studies on compounds found to have effect on the disease to be treated. In general, it is contemplated that an effective antipsychotic amount of the active ingredient would be from about from 0.00002 mg/kg to about 0.009 mg/kg of body weight, in particular from about 0.0001 to about 0.009 mg/kg of body weight, preferably from about 0.0003 mg/kg to about 0.004 mg/kg body weight, more preferably from about 0.0004 mg/kg to about 0.002 mg/kg body weight. The required dose may advantageously be administered as one, two, three or more times at appropriate intervals throughout the day.

According to another aspect, the present invention relates to a method for the treatment of diseases of the central or peripheral nervous system, comprising administering to warm-blooded animals, in particular humans, in need of such treatment an effective amount of the ocaperidone salt as described above.

According to the invention, the term treatment denotes curative, symptomatic, and preventive treatment. Such ocaperidone salts, compositions comprising the same, or treatment can be implemented alone or in combination with other active ingredients, compositions or treatments. Moreover, it can correspond to treatment of chronic or acute disorders.

Formulations of the present invention suitable for oral administration may be presented as discrete units such as capsules, cachets or tablets each containing a predetermined amount of active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus or paste.

A tablet may be made by compression or moulding, optionally with one or more accessory ingredients. Compressed tablets may be prepared by compressing in a suitable machine the active ingredient in a free-flowing form such as powder or granules, optionally mixed with a binder, lubricant, inert diluents, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parental administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example saline or water-for-injection, immediately prior to use.

In a particular embodiment, composition of the invention is suitably formulated for a topical administration in the mouth, for example buccally or sublingually. In this particular embodiment, the ocaperidone salt of the invention can be in a crystalline and/or amorphous form, it is more particularly in an amorphous state. Said buccal or sublingual formulations include lozenges comprising the active ingredient in a flavored basis such as sucrose and acacia or tragacanth, pastilles comprising the active ingredient in a basis such as gelatin and glycerin or sucrose and acacia or any other form adapted to immediate release of the active ingredient in the mouth.

Extemporaneous injection solutions and suspensions may be prepared from sterile powders, granules and tablets of the kind previously described.

Formulations for rectal administration may be presented as a suppository with the usual carriers, such as cocoa butter or polyethylene glycol.

The relatively non-hygroscopic nature and the high solubility of the salts of ocaperidone of the invention render them particularly suitable for administration in liquid and solid form. Preferred unit dosage formulations are those containing an effective dose, as herein before recited, or an appropriate fraction thereof, of the active ingredient. It should be understood that in addition to the ingredients particularly mentioned above, the formulations of this invention may include other agents conventional in the art having regard to the type of formulation in question, for example those suitable for oral administration may includes flavoring agents.

### FIGURES

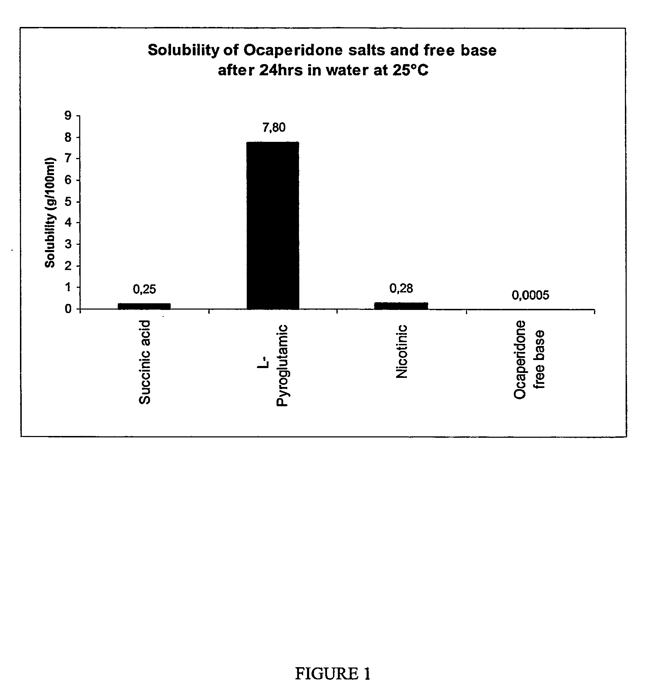

Figure 1 : Ocaperidone free base and salts solubility study in water at 25°C (24 hours) in g/100ml.

The invention is illustrated by the following examples. However, they are representative only and should not be construed as being limiting in any respect.

### EXAMPLES

The powder X-ray diffraction spectrum was measured under the following experimental conditions:

| | | |
|---|---|---|
| DIFFRACTOMETER: | Rigaku MiniFlex | |
| DETECTOR SC-M: | | Scintillator: NaI (T1) |
| | | Window material: Be |
| X-RAY GENERATOR: | Copper anticathode (Lambda = 1,5405) | |
| | | Tube output voltage: 30 kV |
| | | Tube output current: 15 mA |
| | | Kβ suppression filter: Ni-filter |
| GONIOMETER: | Scanning axis: θ/2 θ interlocked | |
| | | 2θ scanning range: -3° to +150° |
| | | Measurement range: +3° to +60° |
| | | Datum angle: 2θ=10° |
| | | Scattering: 4.2 deg. |
| | | Receiving: 0.3 mm |
| | | Scan speed: 2.00 s |
| | | Increment between each measurement: 0.02 deg. |

Experimental data processed using MiniFlex Program Manager Vers. 3.1

NMR spectra were obtained at 300 MHz on a Bruker AV 300 instrument using deuterated solvents. Chemical shifts are given in ppm relative to an internal standard of the solvent (ex: 4.79 for D₂O).

### EXAMPLE 1

### Preparation of Ocaperidone succinic acid salt

100 mg (0.237 mmol) of ocaperidone and 28 mg of succinic acid were dissolved in 15 ml of an ethanol/THF (9/1) solution. The precipitated crystalline product was filtered, washed with cold ethanol and dried. The ocaperidone succinate salt (1/1) thus obtained melts at 184-185°C. ¹H NMR (300 MHz, CD₃OD + D₂O) : 8.83 (d, 1H), 7.90 (dd, 1H), 7.77 (d, 1H), 7.38 (dd, 1H), 7.30-7.15 (m, 2H), 3.82 (brd, 2H), 3.64-3.52 (m, 1H), 3.37-3.29 (m, 3H), 3.23-3.14 (m, 2H), 2.58 (s, 3H), 2.53 (s, 3H), 2.46 (s, 4H), 2.46-2.38 (m, 2H), 2.33-2.18 (m, 2H). Analysis calculated for the formula : C₂₈H₃₁FN₄O₆ : C, 62.44 ; H, 5.54 ; N, 11.09. Found : C, 62.24 ; H, 5.80 ; N, 11.40.

### EXAMPLE 2

### Preparation of Ocaperidone pyroglutamic acid salt

1 g (2.37 mmol) of ocaperidone and 357 mg of L-pyroglutamic acid were dissolved in 10 ml of boiling THF. The precipitated crystalline product was filtered, washed with cold THF and dried. The ocaperidone pyroglutamate (1/1) thus obtained melts at 173-175°C. ¹H NMR (300 MHz, CD₃OD + D₂O) : 8.79 (d, 1H), 7.89 (dd, 1H), 7.74 (d, 1H), 7.35 (dd, 1H), 7.21 (t, 1H), 7.18 (dt, 1H), 4.06 (dd, 1H), 3.82 (brd, 2H), 3.56 (brt, 1H), 3.40-3.25 (m, 4H), 3.20-3.12 (m, 2H), 2.56 (s, 3H), 2.50 (s, 3H), 2.47-2.36 (m, 3H), 2.35-2.20 (m, 3H), 2.08-1.95 (m, 1H). Analysis calculated for the formula : C₂₉H₃₂FN₅O₅: C, 63.37 ; H, 5.67 ; N, 12.74. Found : C, 62.98 ; H, 5.91 ; N, 12.84.

### EXAMPLE 3

### Preparation of Ocaperidone fumaric acid salt

Following example 1, using fumaric acid instead of succinic acid, ocaperidone fumaric acid salt was isolated. m.p. = 204-206° C. ¹H NMR (300 MHz, CD₃OD + D₂O) : 8.81 (d, 1H), 7.86 (dd, 1H), 7.74 (d, 1H), 7.36 (d, 1H), 7.25-7.10 (m, 2H), 6.56 (s, 2H), 3.80 (brs, 2H), 3.54 (brs, 1H), 3.35-3.25 (m, 4H), 3.17-3.07 (m, 2H), 2.54 (s, 3H), 2.50 (s, 3H), 2.46-2.34 (m, 2H), 2.29-2.12 (m, 2H). Analysis calculated for the formula : C₂₈H₂₉FN₄O₆: C, 62.68 ; H, 5.45 ; N, 10.44. Found: C, 62.21 ; H, 5.77 ; N, 11.25.

### EXAMPLE 4

### Preparation of Ocaperidone tartaric acid salt

Following example 1, using tartaric acid instead of succinic acid, ocaperidone tartaric acid salt was isolated. m.p. = 200-202° C. ¹H NMR (300 MHz, CD₃OD + D₂O) : 8.79 (d, 1H), 7.83 (m, 1H), 7.75 (d, 1H), 7.36 (d, 1H), 7.25-7.10 (m, 2H), 4.32 (s, 4H), 3.85 (brs, 2H), 3.52 (brs, 2H), 3.35-3.25 (m, 4H), 3.17-3.07 (m, 2H), 2.53 (s, 3H), 2.50 (s, 3H), 2.46-2.34 (m, 2H), 2.29-2.12 (m, 2H).

### EXAMPLE 5

### Preparation of Ocaperidone N-(2-carboxyphenyl)-glycine acid salt

Following example 2, using N-(2-carboxyphenyl)-glycine acid instead of L-pyroglutamic acid, ocaperidone N-(2-carboxyphenyl)-glycine acid salt was isolated. m.p. = 184-186° C. ¹H NMR (300 MHz, CD₃OD + D₂O) : 8.80 (d, 1H), 7.86 (dd, 1H), 7.73 (m, 2H), 7.34 (dd, 1H), 7.26-7.10 (m, 3H), 6.54-6.43 (m, 2H), 3.74 (brs, 2H), 3.66 (s, 2H), 3.50 (brs, 1H), 3.17-3.07 (m, 2H), 2.53 (s, 3H), 2.48 (s, 3H), 2.43-2.32 (m, 2H), 2.25-2.13 (m, 2H). Analysis calculated for the formula : C₃₃H₃₄FN₅O₆ : C, 64.38; H, 5.57; N, 11.38. Found: C, 64.10 ; H, 5.38 ; N, 12.13.

### EXAMPLE 6

### Preparation of diglycolic acid salt

Following example 2, using diglycolic acid instead of L-pyroglutamic acid, ocaperidone diglycolic acid salt was isolated. m.p. = 158-160° C. ¹H NMR (300 MHz, CD₃OD + D₂O) : 8.91 (d, 1H), 7.97 (dd, 1H), 7.85 (dd, 1H), 7.35-7.23 (m, 2H), 4.09 (s, 4H), 3.96 (brs, 2H), 3.63 (brs, 1H), 3.43-3.36 (m, 4H), 3.29-3.22 (m, 2H), 2.64 (s, 3H), 2.61 (s, 3H), 2.57-2.46 (m, 2H), 2.37-2.22 (m, 2H). Analysis calculated for the formula : C₂₈H₃₁FN₄O₇ : C, 60.64 ; H, 5.63 ; N, 10.10. Found : C, 61.26 ; H, 5.22 ; N, 10.46.

### EXAMPLE 7

### Preparation of Ocaperidone galactaric acid salt

Following example 2, using galactaric acid instead of L-pyroglutamic acid, ocaperidone galactaric acid salt was isolated. m.p. = 180-182° C. ¹H NMR (300 MHz, CD₃OD + D₂O) : 8.81 (d, 1H), 7.87 (dd, 1H), 7.76 (d, 1H), 7.38 (dd, 1H), 7.23 (t, 1H), 7.18 (dt, 1H), 4.15 (s, 1H), 3.86 (s, 1H), 3.84 (brs, 2H), 3.55 (brs, 1H), 3.33-3.26 (m, 2H), 3.18-3.10 (m, 2H), 2.56 (s, 3H), 2.51 (s, 3H), 2.46-2.35 (m, 2H), 2.27-2.09 (m, 2H).

### EXAMPLE 8

### Preparation of Ocaperidone orotic acid salt

Following example 2, using orotic acid instead of L-pyroglutamic acid, ocaperidone orotic acid salt was isolated. m.p. = 245-246° C. ¹H NMR (300 MHz, CD₃OD + D₂O) : 8.80 (d, 1H), 7.87 (dd, 1H), 7.77 (d, 1H), 7.38 (dd, 1H), 7.25 (t, 1H), 7.18 (brt, 1H), 6.04 (s, 1H), 3.75 (brs, 2H), 3.53 (brs, 1H), 3.18-3.10 (m, 2H), 2.55 (s, 3H), 2.51 (s, 3H), 2.45-2.35 (m, 2H), 2.26-2.10 (m, 2H).

### EXAMPLE 9

### Preparation of Ocaperidone hippuric acid salt

Following example 2, using hippuric acid instead of L-pyroglutamic acid, ocaperidone hippuric acid salt was isolated. m.p. = 163-165° C. ¹H NMR (300 MHz, CD₃OD + D₂O) : 8.77 (d, 1H), 7.82 (dd, 1H), 7.76 (m, 2H), 7.62 (d, 1H), 7.41 (m, 1H), 7.37-7.29 (m, 3H), 7.13-7.06 (m, 2H), 3.87 (s, 1H), 3.59 (brd, 2H), 3.37 (m, 1H), 3.04 (s, 4H), 2.94 (t, 2H), 2.50 (s, 3H), 2.47 (s, 3H), 2.26 (dd, 2H), 2.20-2.06 (m, 2H).

### EXAMPLE 10

### Preparation of Ocaperidone nicotinic acid salt

Following example 2, using nicotinic acid instead of L-pyroglutamic acid, ocaperidone nicotinic acid salt was isolated. m.p. = 160-161° C. ¹H NMR (300 MHz, CD₃OD + D₂O) : 8.93 (d, 1H), 8.82 (d, 1H), 8.53 (dd, 1H), 8.22 (brd, 1H), 7.92 (dd, 1H), 7.75 (d, 1H), 7.44 (t, 1H), 7.40 (dt, 1H), 7.23 (t, 1H), 7.17 (dt, 1H), 3.89 (brs, 2H), 3.61 (brs, 1H), 3.45-3.33 (m,3H), 3.26-3.17 (m, 2H), 2.62 (s, 3H), 2.49 (s, 3H), 2.47-2.40 (m, 2H), 2.38-2.23 (m, 2H).

### EXAMPLE 11

### General procedure for the preparation of amorphous salt

A 1:1 mixture of ocaperidone (0.5 mmol) and the desired acid (0.5 mmol) was added into 8ml of water and 2ml of THF to get an homogeneous solution. The obtained mixture was freezed in a cold bath (at -78°C) and put to lyophilize overnight to obtain a white solid.

### EXAMPLE 12

### Solubility of ocaperidone salts in water

Ocaperidone pyroglutamic acid salt of example 2 presents a higher solubility in water than the other salts described in the examples 1, 3-10 and ocaperidone free base.
In particular, the solubilities of ocaperidone salts were measured as follows:
UV/visible spectrophotometer: Agilent 8453E
Balance: Mettler Toledo AX205
Volumetric flask (5ml, 10 ml, 20ml, 100ml)
Mechanical pipettes : Biohit (m1000, m200)
Thermostat: Lauda E112T
Magnetic stirrer : Heidolph MR3001K

A suspension of 100mg of crystalline ocaperidone salt or ocaperidone free base in 1 ml HPLC grade pure water (pH = 6) was prepared. The suspension was kept at fixed and controlled temperature (ex : 25°C) under magnetic stirring for 24 hours. A sample was taken, filtered and the solubility of the ocaperidone salts or ocaperidone free base was measured after 1 h and 24 h. Results are given in g/100ml.

The obtained results are presented in figure 1. These results show the unexpectedly high solubility of ocaperidone pyroglutamic salt.

## Claims

1. A salt of ocaperidone, wherein it presents an acid moiety selected from pyroglutamic acid, N-(2-carboxyphenyl)-glycine acid, diglycolic acid, orotic acid, galactaric acid, nicotinic acid and hippuric acid.

2. The salt according to claim 1, in amorphous and/or crystalline forms, including all polymorphs of said salt.

3. The salt according to claim 1 or 2, wherein the acid moiety is in the D-, L- form, if existent, or mixture thereof.

4. The salt according to anyone of the preceding claims, wherein the acid moiety is selected from pyroglutamic acid, nicotinic acid and hippuric acid.

5. A L-pyroglutamic acid addition salt of ocaperidone of the following formula (II) :

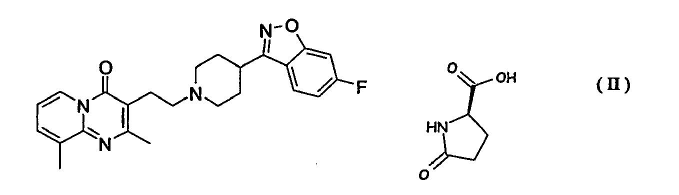

6. A pharmaceutical composition comprising at least one salt as defined in one of the preceding claims, and a pharmaceutically acceptable vehicle or support.

7. The pharmaceutical composition according to claim 6, intended to treat diseases of the central or peripheral nervous system, especially central nervous diseases, including psychosis.

8. The pharmaceutical composition according to claim 7, intended to treat schizophrenia, obsessive compulsive disorder (OCD), bipolar depression, anxiety, mania, or Tourette syndrome.

9. The pharmaceutical composition according to anyone of claims 6-8, which is suitable for a buccal or sublingual formulation.

10. A method for preparing a salt as defined in any of the preceding claims 1-5, including dissolution of ocaperidone and an organic acid selected from pyroglutamic acid, N-(2-carboxyphenyl)-glycine acid, diglycolic acid, orotic acid, galactaric acid, nicotinic acid and hippuric acid, preferably in stoichiometric proportion, and advantageously in an organic solvent, and isolation of the obtained salt.

## Patentansprüche

1. Ocaperidonsalz, enthaltend eine Säuregruppe ausgewählt aus Pyroglutaminsäure, N-(2-Carboxyphenyl)glycinsäure, Diglykolsäure, Orotsäure, Schleimsäure, Nicotinsäure und Hippursäure.

2. Salz nach Anspruch 1 in amorphen und/oder kristallinen Formen einschließlich aller Polymorphe dieses Salzes.

3. Salz nach Anspruch 1 oder 2, wobei die Säuregruppe in der D-, L-Form, falls existierend, oder einer Mischung davon vorliegt.

4. Salz nach einem der vorhergehenden Ansprüche, wobei die Säuregruppe ausgewählt ist aus Pyroglutaminsäure, Nicotinsäure und Hippursäure.

5. L-Pyroglutaminsäureadditionssalz von Ocaperidon mit der folgenden Formel (II):

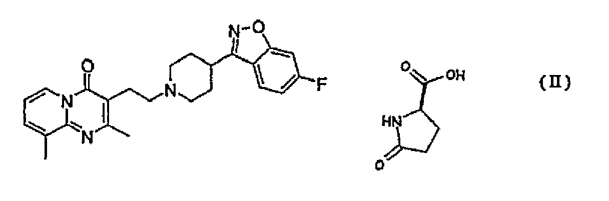

6. Pharmazeutische Zusammensetzung, enthaltend wenigstens ein wie in einem der vorhergehenden Ansprüche definiertes Salz und ein pharmazeutisch unbedenkliches Vehikel oder einen pharmazeutisch unbedenklichen Träger.

7. Pharmazeutische Zusammensetzung nach Anspruch 6 zur Behandlung von Erkrankungen des zentralen oder peripheren Nervensystems, insbesondere zentralnervösen Erkrankungen, einschließlich Psychose.

8. Pharmazeutische Zusammensetzung nach Anspruch 7 zur Behandlung von Schizophrenie, Zwangsneurose (Obsessive Compulsive Disorder, OCD), manischdepressiver Krankheit, Angst, Manie oder Tourette-Syndrom.

9. Pharmazeutische Zusammensetzung nach einem der Ansprüche 6 bis 8, die für eine bukkale oder sublinguale Formulierung geeignet ist.

10. Verfahren zur Herstellung eines wie in einem der vorhergehenden Ansprüche 1 bis 5 definierten Salzes, welches das Auflösen von Ocaperidon und einer aus Pyroglutaminsäure, N-(2-Carboxyphenyl)-glycinsäure, Diglykolsäure, Orotsäure, Schleimsäure, Nicotinsäure und Hippursäure ausgewählten organischen Säure, vorzugsweise in einem stöchiometrischen Verhältnis, und vorteilhafterweise in einem organischen Lösungsmittel, und die Isolierung des erhaltenen Salzes umfaßt.

## Revendications

1. Sel d'ocapéridone **caractérisé en ce qu'**il présente un motif acide choisi parmi l'acide pyroglutamique, l'acide N-(2-carboxyphényl)glycine, l'acide diglycolique, l'acide orotique, l'acide galactarique, l'acide nicotinique et l'acide hippurique.

2. Sel selon la revendication 1, sous des formes amorphes et/ou cristallines, y compris tous les polymorphes dudit sel.

3. Sel selon la revendication 1 ou 2, **caractérisé en ce que** le motif acide est sous la forme D, L, si elle existe, ou sous la forme d'un mélange de celles-ci.

4. Sel selon l'une quelconque des revendications précédentes, **caractérisé en ce que** le motif acide est choisi parmi l'acide pyroglutamique, l'acide nicotinique et l'acide hippurique.

5. Sel d'addition d'acide L-pyroglutamique de l'ocapéridone de la formule (II) suivante :

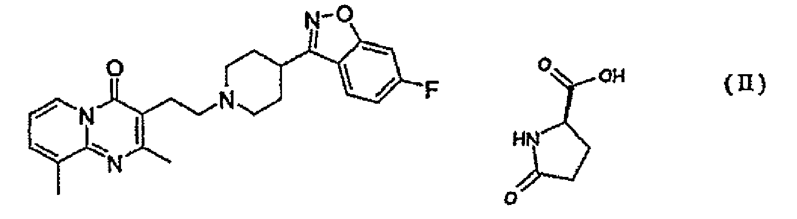

6. Composition pharmaceutique comprenant au moins un sel, tel que défini dans l'une quelconque des revendications précédentes, et un véhicule ou un support pharmaceutiquement acceptable.

7. Composition pharmaceutique selon la revendication 6, destinée à traiter des maladies du système nerveux central ou périphérique, en particulier des maladies du système nerveux central, y compris la psychose.

8. Composition pharmaceutique selon la revendication 7, destinée à traiter la schizophrénie, le trouble obsessif compulsif (TOC), la dépression bipolaire, l'anxiété, la manie ou le syndrome de Tourette.

9. Composition pharmaceutique selon l'une quelconque des revendications 6 à 8, **caractérisée en ce qu'**elle convient à une formulation orale ou sublinguale.

10. Méthode de préparation d'un sel, tel que défini dans l'une quelconque des revendications précédentes 1 à 5, comprenant la dissolution de l'ocapéridone et d'un acide organique choisi parmi l'acide pyroglutamique, l'acide N-(2-carboxyphényl)glycine, l'acide diglycolique, l'acide orotique, l'acide galactarique, l'acide nicotinique et l'acide hippurique, de préférence, dans une proportion stoechiométrique et, avantageusement, dans un solvant organique, et l'isolement du sel obtenu.
